# DEMANDE DE BREVET EUROPEEN

(11) **EP 1 655 048 A1**
(43) Date de publication de la demande: **10.05.2006**
(21) Numéro de dépôt: 04405675.2
(22) Date de dépôt: 03.11.2004
(51) Int. Cl.: A61M 5/32

(54) **Dispositif multifonctionnel pour seringues et methode d'utilisation de ce dispositif**

(71) Demandeur: Biofluid Systems Société Anonyme, 1260 Nyon (CH)
(72) Inventeur: Rochat, Jean-Denis, 1272 Genolier (CH); Marti, René-Andréas, 4153 Reinach (CH)
(74) Mandataire: Savoye, Jean-Paul

(57) **Abrégé**

Dispositif (1) multifonctionnel pour seringues, comprenant une structure (2) supportée par au moins une surface d'appui (3) pourvue d'un moyen d'accrochage (4). Ladite structure comprend également une pluralité de logements (10) destinés chacun à recevoir une aiguille (40) d'une seringue et à maintenir cette dernière par le biais d'une embase (41). Fixée à demeure à l'aiguille, cette embase (41) forme avec cette dernière un tout détachable du corps de la seringue. Les logements (10) sont chacun dotés d'une ouverture (11) située en une partie frontale (5) du dispositif, lequel comprend des moyens de blocage (26, 27, 30) desdites embases (41) empêchant tout mouvement de ces dernières par rapport à leur logement (10).

## Description

La présente invention a pour objet un dispositif multifonctionnel pour seringues, notamment pour seringues à embout luer ou luer lock utilisées en salles d'opération et pour toute injection, ainsi qu'une méthode d'utilisation de ce dispositif.

Au cours d'interventions pratiquées dans les salles d'opérations, différentes doses de médicaments sont administrées aux patients au moyen de seringues. Selon la dose des substances administrée, la fréquence des injections ou la précision que requièrent certains dosages, il est courant d'utiliser des seringues de différents diamètres ou de différents types que l'on remplira et disposera par avance sur un plateau dans l'ordre de leur utilisation.

Le remplissage des seringues se fait en prélevant le médicament désiré dans des vials ou ampoules après en avoir percé une membrane élastique à l'aide de l'aiguille. Pour ce faire, il faut assembler une aiguille sur le corps de la seringue. Cet assemblage se fait par le biais d'une embase solidaire de l'aiguille, laquelle embase est insérée dans l'embout luer du corps de la seringue. Cette jonction embase-embout peut être du type luer ou luer-lock. Ces types de connexion présentent des cônes normalisés bien connus de l'homme du métier. Les embouts luer-lock se différentient des embouts luer par le fait qu'ils comportent un pas de vis alors que les embouts luer s'assemblent par simple emboîtement dans l'embase de l'aiguille.

En cours d'opération, les injections se font sans aiguille; l'injection des substances s'opérant au travers d'accès veineux (cathéters) directement reliés au patient. Cette méthode permet d'administrer à ce dernier autant de substances qu'il est nécessaire, sans devoir à chaque fois le piquer. Dans ce cas, les aiguilles ne servent qu'au remplissage des seringues. Une fois celles-ci préparées, les aiguilles peuvent être enlevées du corps de la seringue puis jetées dans un container spécialement prévu pour les déchets médicaux pointus ou tranchants.

Lorsqu'elles ne sont pas utilisées, les seringues sont par exemple obturées par des bouchons stériles pour éviter tout problème de contamination. Or, tout défaut de rigueur dans cette pratique fait encourir au personnel soignant le risque de se piquer accidentellement, de mélanger les bouchons et de brouiller la disposition ordonnée des seringues qui, à plusieurs reprises, sont saisies puis redéposées sur le plateau.

Pour résoudre en partie ce problème, il a déjà été suggéré, dans le document US5,099,992, d'utiliser un support à seringues, de forme parallélépipédique, présentant deux faces opposées pourvues d'une multitude de cônes luer et d'ouvertures circulaires. Ces cônes luer permettent de recevoir des douilles, elles-mêmes percées de deux cônes luer positionnés tête-bêche. L'un de ces cônes servant à accueillir l'embout luer d'une seringue, laquelle peut alors être maintenue verticalement sans son aiguille. Les ouvertures circulaires ménagées dans ce support servant quant à elles au maintient d'ampoules et d'aiguilles les traversant.

Le document US5,311,985 décrit un autre support pour seringues hypodermiques permettant d'éviter tout risque de blessure et de contamination involontaires. Egalement de forme parallélépipédique, ce support comprend un bloc d'une matière spongieuse, stérile et inerte, qui est facilement pénétrable. Dans ce bloc, sont insérés verticalement tous les bouchons des seringues qui seront utiles pour l'opération. La disposition de ces bouchons est libre et peut donc être ordrée selon les convenances de l'anesthésiste. A l'intérieur de la matière spongieuse, le bloc peut renfermer une poche remplie d'un liquide de désinfection destiné à se répandre une fois la poche percée. En outre, le bloc comprend un socle équipé de surfaces adhésives permettant de le fixer rigidement, par exemple sur une table ou sur un chariot. Enfin, pour garder ce support stérile, le bloc est encore recouvert d'un film protecteur qu'il convient de retirer avant utilisation.

Le principal inconvénient de ces genres de supports réside dans le fait qu'ils ne peuvent remplir qu'une seule fonction, à savoir celle de supporter des seringues de n'importe quel diamètre dans un rangement ordré. A chaque fois, il est donc nécessaire de recourir à l'utilisation d'un container spécial pour pouvoir jeter les aiguilles usagées. De plus, la disposition verticale des seringues ne favorise pas les mouvements du médecin lorsqu'il est amené à devoir retirer ou insérer les seringues dans leur support. Certains de ces dispositifs, comme celui du document US 5,099,992, nécessitent de plus le recours à des douilles ou autres pièces de raccord additionnelles qu'il convient de ne pas égarer et de compter en suffisance. En outre, on constatera que de telles pièces risquent fort de se détacher de leur support et d'être emportées par l'embout de la seringue lorsque celle-ci est retirée du support. Ce problème peut également survenir avec les bouchons des seringues lorsque ceux-ci sont insérés dans une matière spongieuse, et davantage encore lorsque cette matière est imbibée d'un liquide.

Le but de la présente invention vise à remédier, au moins en partie, aux inconvénients précités, en suggérant un dispositif pour seringues qui soit multifonctionnel et qui, en particulier, convienne à une utilisation faite en salles d'opération. A ces fins, la présente invention vise également à suggérer une méthode pour l'utilisation de ce dispositif.

A cet effet, la présente invention a pour objet un dispositif multifonctionnel pour seringues selon la revendication 1. Elle a également pour objet une méthode d'utilisation de ce dispositif selon la revendication 8

Les principaux avantages de ce dispositif résident dans le fait que l'objet de la présente invention peut simultanément faire office d'organe de préhension pour pouvoir détacher, d'une manière simple et efficace, les aiguilles des corps des seringues, faire office de support ergonomique pour maintenir ces seringues dans un rangement logique et facile d'accès au sein d'un milieu stérile, et finalement faire office de réceptacle pour la collecte des aiguilles usagées. En outre, les manipulations que requièrent ces différentes fonctions peuvent également se faire d'une seule main, sans approche des doigts près de l'aiguille.

D'autres avantages apparaîtront à la lumière de la description qui va suivre, se référant à un mode de réalisation préféré, pris à titre nullement limitatif et illustré par les figures annexées dans lesquelles:
La figure 1 est une vue en perspective plongeante du dispositif objet de la présente invention.
La figure 2 représente, dans une vue depuis dessous, une perspective de l'objet de la figure 1 sans la lame élastique frontale.
La figure 3 est une vue en coupe de l'objet de l'invention selon le plan vertical passant par la ligne III-III de la figure 1.
La figure 4 est une vue similaire à celle de la figure 3 dans laquelle sont représentées l'aiguille et l'embase d'une seringue.
La figure 5 est une vue similaire à celle de la figure 3, illustrant une variante des moyens de blocage.

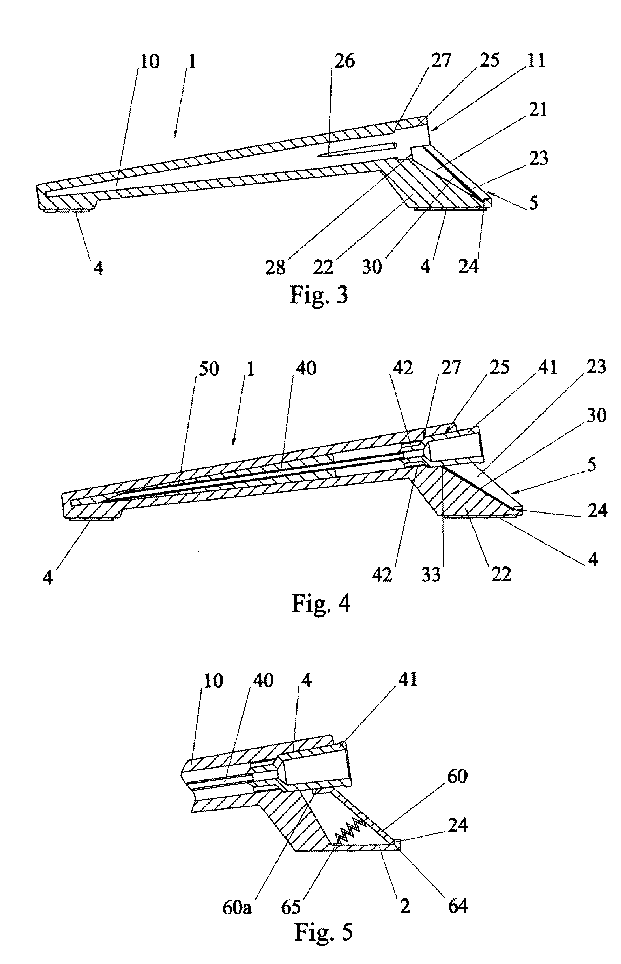

Toute seringue comprend un piston introduit dans un corps pourvu d'une graduation et terminé par un embout. Ces éléments constituent un premier ensemble de pièces qui, généralement, ne sont pas destinées à être séparées les unes des autres. L'aiguille et son embase fixée à une de ses extrémités, constitue un second ensemble de deux pièces indissociables. L'assemblage de ces deux ensembles forme la seringue proprement dite. Cet assemblage est couramment réalisé soit par simple emboîtement en présence d'embouts luer, soit par emboîtement puis par vissage en présence d'embouts de type luer-lock comme décrit précédemment. Seul le second ensemble, constitué de l'aiguille et de son embase, sera illustré et décrit plus en détail en référence à la figure 4.

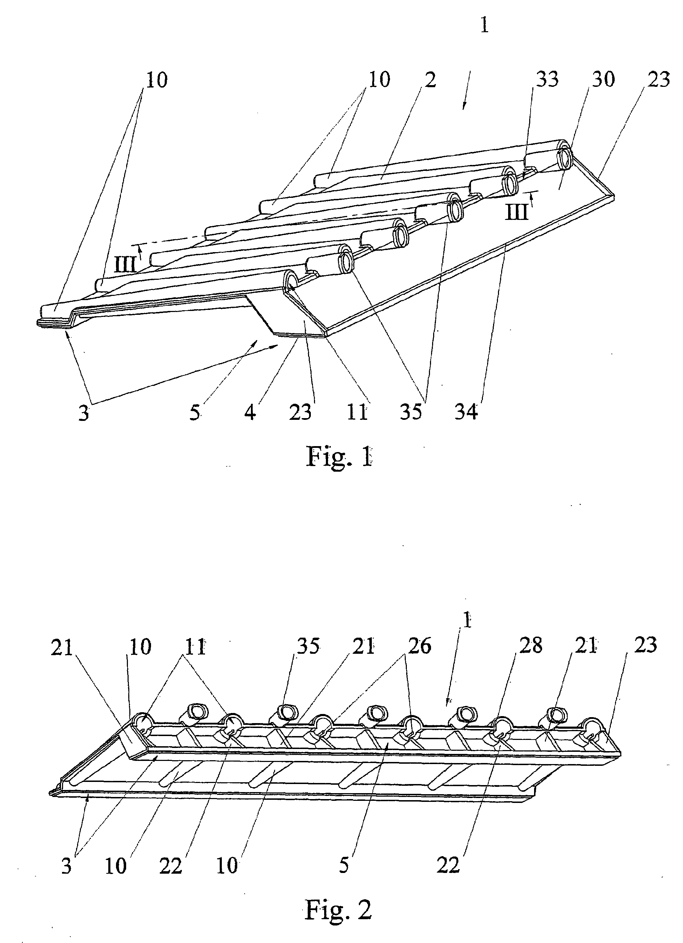

La figure 1 montre un dispositif 1 multifonctionnel pour des seringues telles que celles décrites ci-dessus. Ce dispositif est formé d'une structure 2 relativement plate présentant au moins une surface d'appui 3. Dans le mode de réalisation préféré, sont représentées deux surfaces d'appui 3, chacune équipée d'un moyen d'accrochage 4 permettant temporairement de solidariser le dispositif à un support ou à une surface prévue pour son maintien. Un tel moyen pouvant être typiquement une bande adhésive ou tout organe d'accrochage mécanique, voire magnétique, empêchant le glissement des surfaces d'appui 3 et le déplacement inopiné du dispositif 1.

La structure 2 de ce dispositif supporte également une pluralité de logements 10, chacun destiné à recevoir une aiguille 40 d'une seringue et à maintenir cette dernière par son embase 41 comme illustré à la figure 4. De forme longitudinale, les logements 10 sont de préférence disposés parallèlement les uns aux autres et sont légèrement inclinés vers le haut par rapport au plan que définit les surfaces d'appui 3. L'ergonomie donnée par cette inclinaison permet d'améliorer sensiblement le confort du médecin, notamment dans ses mouvements lorsqu'il est amené à retirer ou à insérer les seringues de leurs logements.

Chaque logement est doté d'une ouverture 11, de forme préférentiellement circulaire, située en une partie frontale 5 du dispositif 1. Dans cette même partie se trouve un organe élastique constitué d'une lame 30, de préférence métallique et tranchante. Cette dernière est avantageusement disposée dans une position non verticale, inclinée en direction des ouvertures 11. Illustrée dans la figure 1 suivant une position dite de repos, cette lame obstrue au moins partiellement les ouvertures 11 des logements 10. Dans cette position, la lame 30 est plane et son arrête supérieure 33 est rectiligne. L'arrête inférieure 34 de cette lame est de préférence glissée et maintenue dans une rainure 24 à la base de la structure 2 comme cela est mieux visible dans la figure 3. Le maintien et le rôle de la lame 30 seront mieux compris à l'étude de la description des figures 2 à 4.

La figure 2 donne une illustration en perspective du dispositif 1 tel que vu depuis le dessous. Dans cette figure, la lame 30 a été retirée pour laisser apparaître les parties de la structure 2 qui s'y trouvaient cachées. Parmi ces parties, on remarque l'existence de premiers appuis frontaux 21 disposés entre les logements 10, de préférence à mi-distance de deux logements adjacents. De seconds appuis frontaux 22, plus courts que les premiers, sont disposés entre les premiers appuis 21, de préférence au droit de chaque ouverture 11. Les extrémités latérales de la structure 2 sont constituées, dans la partie frontale 5, par des faces latérales 23.

Selon le mode de réalisation préféré, ces appuis frontaux sont constitués par des nervures verticales et parallèles les unes aux autres. Les nervures courtes, qui correspondent aux seconds appuis frontaux, ont dans la partie frontale 5 des arrêtes plus inclinées que celles des nervures longues. Dans la position illustrée à la figure 1, la lame 30 repose contre les premiers appuis frontaux 21, à savoir contre les nervures longues, et est maintenue dans le sens transversal par les faces latérales 23.

Le positionnement de la lame 30 par rapport aux appuis frontaux est mieux visible dans la figure 3 qui représente une coupe verticale du dispositif 1 passant par un des logements 10, conformément à la ligne III-III de la figure 1. Sur cette coupe, on peut notamment apercevoir l'intérieur d'un logement 10, lequel est de préférence fermé à son extrémité inférieure, à savoir à l'extrémité qui se trouve à l'opposé de l'ouverture 11. Cette ouverture 11 comprend un cône d'entrée 25 destiné à recevoir l'embout 41 d'une aiguille 40, comme illustré dans la figure 4.

Directement après ce cône d'entrée 25, se trouve au moins une proéminence 26 faisant saillie de la surface intérieure du logement 10. De préférence, deux ou quatre proéminences sont disposées régulièrement autour de cette surface. Lorsqu'une seringue ou du moins une aiguille est insérée dans le logement 10, ces proéminences 26 constituent des premiers moyens de retenue empêchant toute rotation de l'embase 41 autour d'un axe longitudinal imaginaire confondu avec l'aiguille. En effet, les embouts 41 de type luer ou luer-lock sont dotés de quatre ailettes 42 disposées régulièrement à la périphérie terminale de l'embout comme bien visible à la figure 4. Lorsque l'embout 41 est inséré à fond dans le logement 10, en particulier dans le cône d'entrée 25, les proéminences 26 empêchent alors le pivotement de l'embout en bloquant la rotation des ailettes 42.

Comme bien visible sur la figure 3, l'extrémité intérieure du cône d'entrée 25 comprend en outre un épaulement 27. Le cône d'entrée et/ou cet épaulement constituent des seconds moyens de retenue limitant l'enfoncement des aiguilles dans leur logement. En outre, cette figure montre bien l'obstruction partielle de l'ouverture 11 par la lame 30, en particulier par sa partie supérieure située directement en dessous de l'arrête supérieure 33. Cette partie de la lame 30 se trouve en face d'une échancrure 28, également bien visible sur la figure 2. cette échancrure 28 apporte un dégagement qui pourra être investi par la lame dans une position de travail, comme cela va être décrit à présent.

Dans le but d'empêcher tout mouvement des embases, et de ce fait tout mouvement des aiguilles, par rapport à leur logement, il convient également d'empêcher tout retrait de ces dernières une fois insérées dans leur logement. Cette situation est illustrée à la figure 4, laquelle représente notamment l'interaction, en position de travail, de la lame 30 avec l'embase 41 d'une aiguille 40. Dans cette position, la portion de lame se situant au droit de l'ouverture 11 n'est plus rectiligne mais se cintre en direction du second appui frontal 22 qui se trouve au droit de cette même ouverture. Cet appui frontal 22 forme alors une butée pour cette portion de la lame 30. Ainsi, cette lame 30, plus précisément son arrête supérieure 33, ondule entre deux premiers appuis frontaux 21 encadrant un logement 10 occupé par une aiguille, tout en restant plane ou rectiligne de part et d'autre de ces appuis si aucun autre logement 10 n'est occupé par une aiguille.

Pour que la lame 30 soit tranchante, son arrête supérieure 33 est biseautée, de préférence en direction des ouvertures 11. Grâce à l'inclinaison, à la souplesse et à la forme biseautée de cette lame, cette dernière d'une part admet facilement l'introduction d'une aiguille d'une seringue jusqu'à ce que son embase bute contre les seconds moyens de retenue 25, 27, et d'autre part empêche efficacement tout retrait subséquent de l'aiguille en harponnant la surface extérieure de l'embase 41 au moyen de l'arrête supérieure 33.

L'ensemble formé de l'organe élastique, à savoir la lame 30, des premiers moyens de retenue, à savoir les proéminences 26, et des seconds moyens de retenue constitués par l'épaulement 27 et/ou le cône d'entrée 25, constitue les moyens de blocage des embases lorsque des seringues sont introduites dans les logements 10. Avantageusement, ces moyens de blocage permettent de piéger de façon irrémédiable toute aiguille se trouvant introduite dans un logement 10. De ce fait, tout risque de contamination ou d'accident découlant d'une maladresse dans la manipulation des aiguilles, en sera limité.

Avantageusement encore, l'organe élastique est de préférence constitué d'une seule lame. Cependant, on notera que l'agencement d'une pluralité d'organes élastiques disposés au droit de chaque ouverture 11 pourrait être possible. Sans exclure l'utilisation d'un autre matériau, on préférera utiliser une lame métallique de façon à garantir au mieux le tranchant de son arrête supérieure ainsi que les propriétés d'élasticité et de souplesse qu'elle requière. En effet, en s'avançant dans l'échancrure 28 à l'introduction d'une seringue, cette lame doit pouvoir se déformer pour libérer pleinement l'ouverture 11 tout en essayant de reprendre sa position d'origine et d'obliger l'arrête supérieure 33 à pénétrer quelque peu dans l'embase 41 pour la bloquer. On mentionnera également qu'il pourrait être possible de substituer la lame par un autre organe élastique, comme par exemple une succession d'aiguilles flexibles.

Avantageusement, il est encore prévu que les logements 10 soient partiellement remplis d'un gel ou de mousse à basse densité 50 de façon à ce qu'au moins l'extrémité biseautée de l'aiguille puisse y être complètement noyée. Ce gel ou cette mousse aura pour principale fonction d'obstruer l'aiguille par cette extrémité afin de rendre étanche cette portion de seringue.

Avantageusement, l'obstruction de l'aiguille 40 par le gel ou la mousse 50 permet également de maintenir le conduit de cette dernière dans un milieu parfaitement stérile. Ainsi, l'anesthésiste ou l'infirmière pourra utiliser ces aiguilles comme bouchon pour les corps des seringues qui devront être reposées dans leurs logements 10 en vue d'un usage répété au cours de la même opération.

Avantageusement encore, on veillera en outre à préserver un espacement suffisant entre ces logements de façon à pouvoir y insérer et retirer aisément des seringues de n'importe quel diamètre. On remarquera que, grâce à la saillie permanente que fait l'embout 41 hors de l'ouverture 11 lorsqu'une aiguille est introduite dans un logement, le personnel soignant est immédiatement renseigné sur la présence d'une aiguille dans le dispositif 1 et sur le nombre de logements encore disponibles. Aussi, ces logements auront de préférence une forme conique et seront suffisamment longs pour pouvoir y loger des aiguilles de grande dimension.

Des réceptacles luer 35 placés entre les logements 10 permettent aussi de recevoir des seringues non munies d'aiguilles. Dans ce cas les réceptacles ne formerons que bouchon au seringues.

A titre de variantes, les moyens de blocage des embases 41 des aiguilles 40 pourraient présenter une autre forme que celle de la lame 30. C'est ainsi que l'on pourrait avoir un élément en forme d'aiguille (non représentée) maintenue en position écartée de la surface de butée 22 par un ressort de rappel. Cette aiguille serait appliquée contre la surface de butée 22 lorsqu'une aiguille 40 serait introduite dans un logement 10, en sorte que sa pointe pénétrerait dans l'embase 41 si on tentait de la ressortir de ce logement 10.

On pourrait aussi envisager un bras 60 (figure 5) monté oscillant par son extrémité inférieure 64 dans la rainure 24 à la base de la structure 2, à l'encontre de la pression d'un ressort de rappel 65. L'autre extrémité de ce bras est munie d'une surface de frottement 60a conformée pour épouser une partie de la surface externe de l'embase 41. Ainsi, lors de l'introduction de l'aiguille 40, la surface de frottement 60a est appliquée par le ressort de rappel 65 contre l'embase 41. Tout mouvement tendant à extraire l'aiguille 40 du logement 10 a pour effet d'entraîner le bras 60 dans le sens des aiguille de la montre et de provoquer un effet coincement, empêchant la sortie de l'aiguille 40. Plus on tire, plus la force de coincement augmente.

L'objet de la présente invention se réfère également à une méthode d'utilisation du dispositif 1, laquelle comprend les étapes suivantes:

Premièrement, on remplit chaque seringue, au moyen de leur aiguille, du liquide (médicament) destiné à être injecté au patient via un cathéter (non illustré) au moment voulu. On introduit ensuite chaque seringue dans un ordre choisi, à fond dans les logements du dispositif avec leur aiguille.

Le moment d'injecter venu, en reprenant la seringue, l'aiguille se séparera de la seringue et restera piégée dans le dispositif qui formera bouchon lors de la remise en place de la seringue sur le dispositif. Cette opération s'effectuera à l'aide d'une seule main, dans un premier mouvement de torsion et de retrait. L'effet de torsion permettra soit de dévisser l'embout de la seringue pour le séparer de l'embase 41 dans le cas d'une connexion de type luer-lock, soit de faciliter cette même séparation en présence d'une seringue pourvue d'un embout luer. Durant cette opération, l'aiguille 40 reste piégée avec son embase 41 dans ledit logement 10, essentiellement grâce aux moyens de blocage 26 et 30.

Après avoir injecté au moins une partie du liquide dans le cathéter, on assemblera le corps de la seringue à son aiguille par le biais de son embase. Pour ce faire, on effectuera, dans un second mouvement, l'inverse du premier mouvement.

Si l'usage de la seringue n'est pas terminé, on répétera successivement les deux étapes précédentes.

Enfin, une fois l'opération terminée, on jettera dans une poubelle le dispositif avec toutes les aiguilles s'y trouvant piégées, sans devoir faire usage d'un container particulier pour récolter les aiguilles usagées (déchets médicaux infectieux).

Avantageusement, on remarquera que cette méthode s'appuie sur une séquence de manipulations dont le nombre d'opérations est inférieur à ceux que nécessitent les méthodes traditionnelles. En effet, non seulement l'opération visant à jeter les aiguilles dans un container particulier est supprimée, mais d'autre part il n'est plus nécessaire ni d'adjoindre ni de manipuler un bouchon ou une pièce particulière pour maintenir la seringue dans un support et la conserver à la fois dans un milieu stérile à l'abri de toute contamination. A cela, on remarquera également que la manipulation visant à prendre la seringue pour la retirer de son support permet en même temps d'effectuer la séparation de l'aiguille du corps de la seringue dans des conditions parfaitement stérile et à l'abri de tout risque de piqûre pour l'anesthésiste.

## Revendications

1. Dispositif (1) multifonctionnel pour seringues, comprenant une structure (2) supportée par au moins une surface d'appui (3) pourvue d'un moyen d'accrochage (4), une pluralité de logements (10) destinés chacun à recevoir une aiguille (40) d'une seringue et à maintenir cette dernière par le biais d'une embase (41), laquelle embase (41) fixée à demeure à l'aiguille (40) forme avec cette dernière un tout détachable du corps de la seringue, lesdits logements (10) étant chacun dotés d'une ouverture (11) située en une partie frontale (5) du dispositif, **caractérisé en ce qu'**il comprend des moyens de blocage (25, 26, 27, 30) desdites embases (41) empêchant tout mouvement de ces dernières par rapport à leur logement (10).

2. Dispositif selon la revendication 1, **caractérisé en ce que** lesdits moyens de blocage (26, 27, 30) sont constitués de premiers moyens de retenue (26) empêchant toute rotation de l'embase autour d'un axe longitudinal confondu avec l'aiguille (40), de seconds moyens de retenue (25, 27) limitant l'enfoncement de l'aiguille (40) et de son embase (41) dans leur logement (10), et d'au moins un organe élastique (30) empêchant tout retrait des embases (41) hors de leurs logements (10).

3. Dispositif selon la revendication 2, **caractérisé en ce que** l'organe élastique (30) est constitué d'une lame métallique tranchante qui, dans une position de repos, obstrue au moins partiellement les ouvertures (11) frontales desdits logements (10) et, dans une position de travail, est susceptible de s'infléchir au droit de chaque ouverture (11).

4. Dispositif selon la revendication 3, **caractérisé en ce que** ladite lame est disposée dans la partie frontale (5) du dispositif (1), selon une position inclinée en direction des ouvertures (11), et présente une arrête supérieure (33) biseautée dans cette même direction.

5. Dispositif selon la revendication 4, **caractérisé en ce qu'**en position de repos, la lame repose obliquement contre des premiers appuis frontaux (21) disposés entre les logements (10), et **en ce qu'**en position de travail, cette lame est retenue par des seconds appuis frontaux (22), plus courts que les premiers et disposés entre ces derniers.

6. Dispositif selon la revendication 2, **caractérisé en ce que** lesdits premiers moyens de retenue (26) sont constitués par au moins une proéminence faisant saillie à l'intérieur de chacun des logements (10), et **en ce que** lesdits seconds moyens de retenue (25, 27) sont constitués par un cône d'entrée (25) et/ou un épaulement (27) ménagé à l'extrémité intérieure du cône d'entrée (25) situé dans l'ouverture (11) de chaque logement (10).

7. Dispositif selon la revendication 1, **caractérisé en ce que** lesdits logements (10) sont partiellement remplis d'un gel (50) ou d'une mousse basse densité permettant la pénétration des aiguilles.

8. Dispositif selon la revendication 1, **caractérisé en ce que** des réceptacles luer (35) placés entre les logements (10) permettent aussi de recevoir des seringues non munies d'aiguilles.

9. Méthode d'utilisation du dispositif selon la revendication 1, **caractérisé en ce qu'**elle comprend les étapes suivantes:
1° préparer un lot de seringues en les introduisant, dans un ordre choisi, à fond dans les logements (10) du dispositif (1) après les avoir chacune remplies, au moyen de leur aiguille (40), d'un liquide destiné à être injecté dans un cathéter à un moment voulu,
2° ledit moment venu, séparer à l'aide d'une seule main, dans un premier mouvement de torsion et de retrait, l'aiguille (40) et le corps de la seringue, laquelle aiguille (40) reste alors piégée avec son embase (41) dans ledit logement (10) grâce aux moyens de blocage (26, 30),
3° après avoir injecté au moins une partie du liquide dans ledit cathéter, assembler le corps de la seringue à son aiguille (40) formant alors un bouchon temporaire par le biais de son embase (41), dans un second mouvement inverse au premier,
4° si l'usage de la seringue n'est pas terminé, répéter les étapes 2° et 3°,
5° jeter le dispositif avec lesdites seringues.
